# EUROPÄISCHE PATENTSCHRIFT

(11) **EP 1 105 082 B1**
(45) Veröffentlichungstag und Bekanntmachung des Hinweises auf die Patenterteilung: **09.06.2004**
(21) Anmeldenummer: 99923360.4
(22) Anmeldetag: 09.06.1999
(51) Int. Cl.: A61K 6/02, C03C 10/00

(54) **GLASKERAMIK FÜR DIE DENTALE RESTAURATION UND VERFAHREN ZU DEREN HERSTELLUNG**
GLASS-CERAMIC MATERIAL FOR DENTAL RESTORATION AND METHOD FOR PRODUCING SAME
VITRO-CERAMIQUE DESTINEE A LA RESTAURATION DENTAIRE ET SON PROCEDE DE PRODUCTION

(30) Priorität: 17.08.1998 CH 168598
(43) Veröffentlichungstag der Anmeldung: 13.06.2001
(73) Patentinhaber: Chemichl AG, 9490 Vaduz (LI)
(72) Erfinder: BEHAM, Gerhard, FL-9495 Triesen (LI)
(74) Vertreter: Liebetanz, Michael, Dipl.-Phys.
(86) Internationale Anmeldenummer: PCT/CH1999/000249
(87) Internationale Veröffentlichungsnummer: WO 2000/010509

(56) Entgegenhaltungen:
- EP-A- 0 272 745
- EP-A- 0 475 528
- EP-A- 0 690 030
- US-A- 5 622 551
- US-A- 5 653 791
- DATABASE WPI Section Ch, Week 7833 Derwent Publications Ltd., London, GB; Class L02, AN 78-59289A XP002112378 & JP 53 078220 A (OSAKA CITY), 11. Juli 1978 (1978-07-11)

## Beschreibung

Die Erfindung betrifft eine Glaskeramik für die dentale Restauration sowie ein Verfahren zu deren Herstellung.

Eine solche Glaskeramik ist aus der US 4,798,536 bekannt. Dieses Patent beschreibt ein bestimmtes Porzellanmaterial, welches als Zahnersatzwerkstoff zum Einsatz kommt. Porzellan als Zahnersatzwerkstoff ist im Prinzip seit langem bekannt, wobei ebenfalls bekannt ist, dass es relativ bruchanfällig ist. Es wurden daher zumeist metallische Unterkonstruktionen verwendet, um die gewünschte Stärke zu erhalten.

Aus der US 4,798,536 ist ein Porzellanmaterial bekannt, welches solche Eigenschaften der Bruchfestigkeit aufweist, dass es in Alleinstellung als Dentalwerkstoff geeignet scheint.

Es hat sich jedoch herausgestellt, dass die im Mund auftretenden Kräfte und Belastungen das Material dennoch in einer Weise belasten können, dass es bricht.

Ausgehend von diesem Stand der Technik liegt der Erfindung die Aufgabe zugrunde, eine Glaskeramik der eingangs genannten Art derart zu verbessern, dass sie bruchfester ist. Zudem ist es Aufgabe der vorliegenden Erfindung, ein Verfahren zu deren Herstellung anzugeben.

Diese Aufgaben werden mit den Merkmalen der Ansprüche 1 bzw. 9 gelöst.

Vorteilhafte Ausgestaltungen der Erfindung sind in den Unteransprüchen gekennzeichnet. Die Erfindung wird nun unter Bezugnahme auf die beigefügten Diagramme beispielhaft näher beschrieben. Es zeigen:

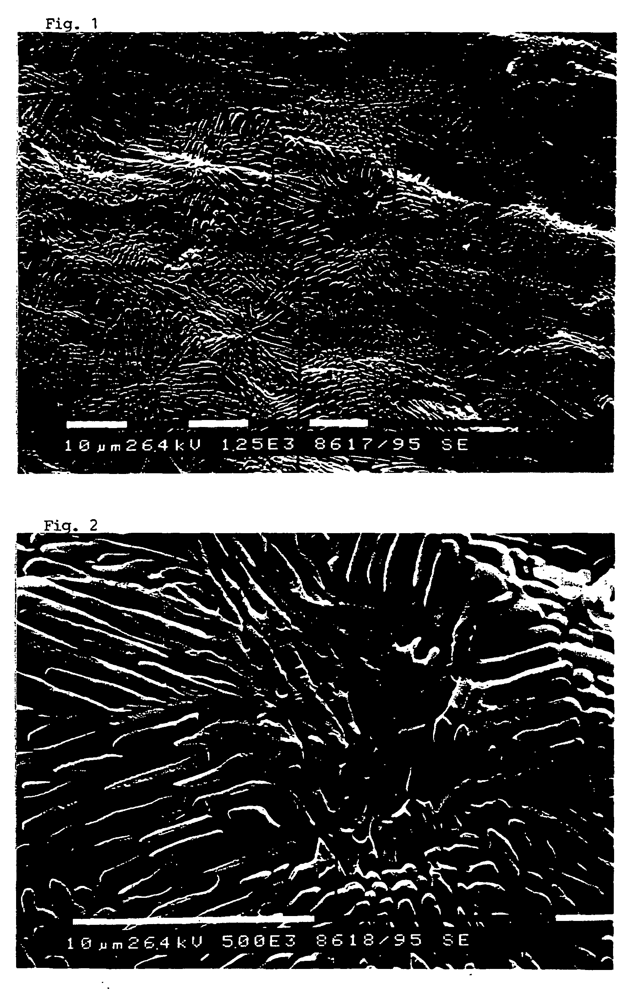

Fig. 1 eine Raster-Elektronen-Mikroskop-Aufnahme, wobei ein Teilstrich der Skala 10 Mikrometer ausmacht, und
Fig. 2 eine Vergrösserung der REM-Aufnahme aus Fig. 1.

Zur Herstellung der semi-transparenten in ihrer Farbe zahnähnlichen Keramik kann von einer Reihe von Feldspatprodukten ausgegangen werden, so z.B. von dem kanadischen oder dem norwegischen Feldspat. Auch andere Ausgangsmaterialien sind einsetzbar. Wichtig ist bei der Auswahl des Feldspats das Kalium-Natrium-Verhältnis. Es ist vorzugsweise Kalifeldspat einzusetzen, der als Mineral immer Natrium enthält. Das Verhältnis Na₂O zu K₂O sollte kleiner als 1 zu 10 sein. Die der Erfindung zugrunde liegende Glaskeramik, d.h. das Endprodukt, entspricht folgender Zusammensetzung in Gewichtsprozenten:

| Material | Gewichtsprozent |
|---|---|
| SiO₂ | 65-75 |
| Al₂O₃ | 8-12 |
| Na₂O | 3-6 |
| K₂O | 8-11 |
| CaO | 1-3 |
| BaO | 0-2 |
| CeO₂ | 0,5-1 |
| TiO₂ | 0,2-0,5 |
| B₂O₃ | 0,5-2 |

Die Rohstoffe sind in Form von Silikaten, Carbonaten oder Oxiden gemischt worden. Die resultierende Rohstoffmischung ist in einem Kaskadentiegel bei ungefähr 1500°C und einem Durchlauf von ungefähr 2 kg/h geschmolzen worden. Die Verweildauer im Tiegel lag bei ungefähr einer Stunde. Die Schmelze kann auch in einem Bereich von 1470 Grad Celsius bis 1550 Grad Celsius und in anderer Form geschmolzen werden.

Die Glasschmelze ist dann aus dem Schmelzofen direkt in Wasser getropft worden und erstarrte spontan zu einem amorphen griessförmigen Glas. Nach dem Trocknen der im Wasser abgeschreckten Glasschmelze erfolgte das Mahlen des grobkörnigen Glases in einer Kugelmühle. Sobald das Mahlgut an der Mahltrommel klebte, ist die Trockenmahlung beendet worden. Das resultierende Glaspulver ist nach dem Mahlen durch ein Sieb < 80 Mikron gesiebt worden. Die mittlere Korngrösse betrug ca. 20 Mikron.

Zur Herstellung von dentalen Restaurationsprodukten aus Glaskeramik ist das Sintern und Tempern des Glases zu den gewünschten Formen und Gegenständen durchgeführt worden. Diese Verfahren werden in der Dentaltechnik zur Herstellung des Zahnersatzes angewandt.

In einer alternativen Vorgehensweise kann das Glaskeramikpulver auch trocken verpresst und anschliessend durch einen Sinter-Temperbrand in einen festen Glaskeramikgegenstand überführt werden. Diese Vorgehensweise wird vorteilhafterweise zur Herstellung von Halbzeug bzw. Rohlingen verwendet, aus denen nach der CAD-CAM-Technik ein individuelles Keramikobjekt vorzugsweise für die zahnärztliche Restauration hergestellt wird.

Der entsprechend obiger Vorgehensweise hergestellte Werkstoff ist eine leucitverstärkte Keramik, mit der vollkeramische Kronen ohne Metallverstärkung hergestellt werden können. Der Leucitanteil der neuen Glaskeramik beträgt mehr als 90 Prozent. Diese Angabe ist aus den REM-Aufnahmen zu entnehmen, da eine analytische quantitative Bestimmung im allgemeinen ungenau ist. Der hohe thermische Ausdehnungskoeffizient von 19,0 * 10⁻⁶ K⁻¹ weist ebenfalls auf einen hohen Leucitgehalt hin. Der Literaturwert des Leucit wird mit 20 bis 22 angegeben.

Die Kristalle bei der US 4,798,536 sind kleiner als 35 Mikron, vorzugsweise kleiner als 5 Mikron, und sind im wesentlichen blattförmig mit einem Verhältnis von Länge zu Breite von 1:1 bis 1:3 und weisen Hauptdimensionen in zwei Richtungen zwischen 1 und 10 Mikron auf.

Bei den Kristallen gemäss der vorliegenden Erfindung ist eine neue nadelförmige und stark verfilzte Struktur entstanden. Die einzelnen Kristalle sind nadel- oder stäbchenförmig mit einer Dicke zwischen 0,3 und 1,5 Mikrometer und weisen eine Länge zwischen 7,5 und 20 Mikrometer auf. Sie orientieren sich vorzugsweise in Gruppen sternförmig ausgehend von einem Keim als Mittelpunkt, von dem aus sich die Leucitkristalle entlang von sternförmigen Bahnen ausbilden. Beim Aufheizen des amorphen Glases bilden sich im Temperaturbereich von 800 bis 900 Grad Celsius die Leucitkristalle. Bereits nach zwei Minuten Haltezeit ist visuell eine deutliche Trübung des ursprünglich transparenten Glases erkennbar. Die Stäbchen sind vorzugsweise 0,5 bis 1 Mikrometer dick bei einer Länge zwischen 8 und 12 Mikrometer. Das Verhältnis von Länge zu Breite beträgt mindestens 5:1 und kann bis zu 15:1 hinaufgehen. Vorzugsweise liegt es im Bereich um 10:1.

Aus diesem Glaskeramikwerkstoff mit einem hohen Anteil an nadelförmigen, in situ erzeugten Leucitkristallen wurden Prüfkörper gesintert, mit der Diamantsäge gesägt und die Dreipunkt-Biegefestigkeit bestimmt. Die gesägten Prüfkörper wiesen eine Biegefestigkeit von 200 MPa auf und bei einer entsprechenden Oberflächenbehandlung wie Glasieren wurden Festigkeiten zwischen 300 und 350 MPa erzielt. Der neue glaskeramische Werkstoff weist daher eine verbesserte Bruchfestigkeit auf und er eröffnet neue Indikationsbereiche für den Einsatz von Vollkeramik in der Dentaltechnik, insbesondere die metallfreie Restauration.

Die Zusammensetzung des Glases ermöglicht nun das gleichzeitige Tempern und Sintern. Die Kristallisation erfolgt relativ spontan und in kurzer Zeit in wenigen Minuten. Nach dem Sintern des Glaspulvers bilden sich die nadelförmigen Kristalle in sternförmiger Anordnung. Das übliche Zweiphasen-Herstellungsverfahren reduziert sich auf einen einzigen Verfahrensschritt, wobei Sintern und Kristallisation simultan erfolgen. Die dabei entstehende nadel- bzw. faserförmige Kristallstruktur verstärkt die Festigkeit gegenüber bisherigen Leucitformen.

Die beiliegenden Fig. zeigen REM-Aufnahmen, bei denen der glaskeramische Werkstoff eine hohe Konzentration ineinander verfilzter Kristallnadeln mit der Dimension von 1 Mikron Durchmesser und 10 Mikron Länge aufweist. Die dabei erreichte Dreipunkt-Biegefestigkeit der leucitverstärkten Glaskeramik ist dabei etwa doppelt so hoch wie bei konventionellen leucitverstärkten Keramiken.

Der Wärmeausdehnungskoeffizient für reine Leucitkristalle liegt ungefähr bei 20 bis 22 * 10⁻⁶ K⁻¹. Mit der Glaskeramik gemäss der Erfindung kann ein Wert von 19 bis 20 * 10⁻⁶ K⁻¹ erreicht werden, was ein Spiegelbild der verbesserten Festigkeit ist. Die Glasmatrix weist einen Koeffizienten auf, der bei ungefähr 10 * 10⁻⁶ K⁻¹ liegt.

Der Stand der Technik kennt relativ inhomogene Leucitkristallverteilungen, die grosse Flächen und damit auch grosse Angriffsflächen für Kräfte bilden. Die Leucitkristalle sind beim Stand der Technik in amorphe Materialien lediglich eingebettet und bilden Festigkeitsinseln in der amorphen Glasphase. Die Erfindung dagegen liefert lange und dünne Kristalle, die in verschiedenste Richtungen orientiert sind und die, wie es in den Zeichnungen erkennbar ist, nicht nur Festigkeitsinseln in der amorphen Glasphase bilden sondern in verbundener Weise um den Keim im Sternmittelpunkt feste Strukturen erstellen, in denen Materialbrüche im Mikrobruchbereich bereits sicher nach kurzen Wegen aufgehalten werden.

## Patentansprüche

1. Glaskeramik für die dentale Restauration mit einem hohen kristallinen Leucitgehalt (mehr als 90 prozent), wobei die Leucitkristalle nadel- oder stäbchenförmig sind, diese eine Dicke zwischen 0,3 und 1,5 Mikrometer aufweisen und deren Länge zwischen 7,5 und 20 Mikrometer beträgt, und wobei die Glaskeramik im wesentlichen ein semitransparentes Material ist, welches umfasst:
| Material | Gewichtsprozent |
|---|---|
| SiO₂ | 67 - 71 |
| Al₂O₃ | 8 - 12 |
| Na₂O | 3 - 5 |
| K₂O | 8 - 10 |
| CaO | 1 - 3 |
| BaO | 0,2 - 2 |
| CeO₂ | 0, 5 - 2 |
| TiO₂ | 0,2 - 1 |
| B₂O₃ | 0,5 - 2 |

2. Glaskeramik nach Anspruch 1, **dadurch gekennzeichnet, dass** die die Leucitkristalle bildenden Nadel oder Stäbchen Bündel von Nadeln oder Stäbchen bilden.

3. Glaskeramik nach Anspruch 2, **dadurch gekennzeichnet, dass** die die Leucitkristalle bildenden Bündel aus Nadeln oder Stäbchen ausgehend von einem Mittelpunkt im wesentlichen sternförmig angeordnet sind.

4. Glaskeramik nach einem der vorstehenden Ansprüche, **dadurch gekennzeichnet, dass** die Leucitkristalle eine Dicke von ungefähr 0,5 bis 1 Mikrometer bei einer Länge zwischen 8 und 12 Mikrometer aufweisen.

5. Glaskeramik nach einem der vorstehenden Ansprüche, **dadurch gekennzeichnet, dass** das semi-transparente Material umfasst:
| Material | Gewichtsprozent |
|---|---|
| SiO₂ | 68 - 71 |
| Al₂O₃ | 9 - 11 |
| Na₂O | 4 - 5 |
| K₂O | 9 - 10 |
| CaO | 1,5 - 2,5 |
| BaO | 0, 5 - 1,5 |
| CeO₂ | 0,5 - 1 |
| TiO₂ | 0,2 - 0,5 |
| B₂O₃ | 0,5 - 2 |

6. Glaskeramik nach einem der vorstehenden Ansprüche, **dadurch gekennzeichnet, dass** dem semi-transparenten Material der Glaskeramik ein Pigment hinzugefügt ist.

7. Glaskeramik nach Anspruch 6, **dadurch gekennzeichnet, dass** das Pigment aus der Gruppe der Chromate, Vanadinate, Manganate und Mischungen von diesen ausgewählt ist.

8. Glaskeramik nach einem der vorstehenden Ansprüche, **dadurch gekennzeichnet, dass** der Wärmeausdehnungskoeffizient ungefähr 19 • 10⁻⁶ K⁻¹ beträgt.

9. Verfahren zur Herstellung einer Glaskeramik für die dentale Restauration nach einem der vorstehenden Ansprüche, **dadurch gekennzeichnet, dass** als Ausgangsprodukte Silikate, Carbonate oder Oxide in einer solchen Zusammensetzung gemischt werden, dass sie nach dem Schmelzvorgang die Zusammensetzung der besagten semi-transparenten Glaskeramik aufweisen, dass die resultierende Rohstoffmischung in einem Kaskadentiegel bei ungefähr 1500°C geschmolzen wird, dass die Glasschmelze aus dem Schmelzofen direkt in Wasser getropft wird, dass das erstarrte Glasmaterial nach dem Trocknen in einer Trockenmahlung gemahlen wird, und dass zur Herstellung von dentalen Restaurationsprodukten aus Glaskeramik das Glaspulver zu den gewünschten Formen und Gegenständen gesintert und gleichzeitig getempert wird oder dass das Glaspulver trocken verpresst und anschliessend durch einen Sinter-Temperbrand in einen festen Glaskeramikgegenstand überführt wird.

10. Verfahren nach Anspruch 9, **dadurch gekennzeichnet, dass** die dentalen Restaurationsprodukte in Form von Kronen oder Inlays auf einem feuerfesten Modell mit einem aus dem Glaspulver und Wasser gebildeten Schlicker geformt und anschliessend bei ungefähr 820 Grad Celsius bei 10 Minuten Haltezeit gesintert und getempert werden oder dass die dentalen Restaurationsprodukte in einem feuerfesten Modell aus einer plastifizierten Glasmasse kaltgepresst und anschliessend bei ungefähr 800 bis 900 Grad Celsius unter Druck gesintert und getempert werden.

## Claims

1. A glass ceramic for dental restoration having a high crystalline leucite content, more than 90 percent, the leucite crystals being in needle or column form, having a thickness of between 0.3 and 1.5 micrometers and having a length of between 7.5 and 20 micrometers, and the glass ceramic substantially being a semi-transparent material which comprises:
| Material | Percent by weight |
|---|---|
| SiO₂ | 67 - 71 |
| Al₂O₃ | 8 - 12 |
| Na₂O | 3 - 5 |
| K₂O | 8 - 10 |
| CaO | 1 - 3 |
| BaO | 0.2 - 2 |
| CeO₂ | 0.5 - 2 |
| TiO₂ | 0.2 - 1 |
| B₂O₃ | 0.5 - 2 |

2. The glass ceramic as claimed in claim 1, **characterized in that** the needles or columns which form the leucite crystals form groups of needles or columns.

3. The glass ceramic as claimed in claim 2, **characterized in that** the groups of needles or columns which form the leucite crystals are arranged substantially in a star shape, starting from a center point.

4. The glass ceramic as claimed in one of the preceding claims, **characterized in that** the leucite crystals have a thickness of approximately 0.5 to 1 micrometer for a length of between 8 and 12 micrometers.

5. The glass ceramic as claimed in one of the preceding claims, **characterized in that** the semi-transparent material comprises:
| Material | Percent by weight |
|---|---|
| SiO₂ | 68 - 71 |
| Al₂O₃ | 9 - 11 |
| Na₂O | 4 - 5 |
| K₂O | 9 - 10 |
| CaO | 1.5 - 2.5 |
| BaO | 0.5 - 1.5 |
| CeO₂ | 0.5 - 1 |
| TiO₂ | 0.2 - 0.5 |
| B₂O₃ | 0.5 - 2 |

6. The glass ceramic as claimed in one of the preceding claims, **characterized in that** a pigment is added to the semi-transparent material of the glass ceramic.

7. The glass ceramic as claimed in claim 6, **characterized in that** the pigment is selected from the group consisting of the chromates, vanadinates, manganates and mixtures thereof.

8. The glass ceramic as claimed in one of the preceding claims, **characterized in that** the coefficient of thermal expansion is approximately 19 • 10⁻⁶ K⁻¹.

9. A process for producing the glass ceramic for dental restoration as claimed in one of the preceding claims, **characterized in that** silicates, carbonates or oxides are mixed as starting materials, in a composition which is such that, after the melting operation, they have the composition of said semi-transparent glass ceramic, **in that** the resultant mixture of raw materials is melted in a cascade crucible at approximately 1500°C, **in that** the molten glass from the melting furnace is added dropwise directly into water, **in that** the solidified glass material, after drying, is milled in a dry milling operation, and **in that** to produce dental restoration products from glass ceramic the glass powder is sintered into the desired shapes and objects and at the same time is annealed, or **in that** the glass powder is dry-pressed and is then converted into a solid glass ceramic object by a sintering/annealing firing operation.

10. The process as claimed in claim 9, **characterized in that** the dental restoration products are shaped in the form of crowns or inlays on a refractory model using a slip formed from the glass powder and water and are then sintered and annealed at approximately 820°C for a holding time of 10 minutes, or **in that** the dental restoration products are cold-pressed in a refractory model from a plasticized glass mass and are then sintered and annealed under pressure at approximately 800 to 900°C.

## Revendications

1. Vitrocéramique pour la restauration dentaire avec un contenu de leucite cristalline de plus que 90 pour cent, où les cristaux de leucite sont en forme d'aiguille ou de baguette, avec une épaisseur entre 0.3 et 1.5 micromètres et avec une longueur entre 7.5 et 20 micromètres, où la vitrocéramique est essentiellement un matériel semi-transparent qui comprend:
| matériel | pour cent en poids |
|---|---|
| SiO₂ | 67 - 71 |
| Al₂O₃ | 8 - 12 |
| Na₂O | 3 - 5 |
| K₂O | 8 - 10 |
| CaO | 1 - 3 |
| BaO | 0.2 - 2 |
| CeO₂ | 0.5 - 2 |
| TiO₂ | 0.2 - 1 |
| B₂O₃ | 0.5 - 2 |

2. Vitrocéramique selon la revendication 1, **caractérisée en ce que** les aiguilles ou baguettes formant les cristaux de leucite forment des faisceaux d'aiguilles ou de baguettes.

3. Vitrocéramique selon la revendication 2, **caractérisée en ce que** les faisceaux d'aiguilles ou de baguettes formant les cristaux de leucite sont arrangés essentiellement dans une forme d'étoile, en partant d'un point central.

4. Vitrocéramique selon l'une des revendications précédentes, **caractérisée en ce que** les cristaux de leucite ont une épaisseur d'environ 0.5 à 1 micromètre pour une longueur comprise entre 8 et 12 micromètres.

5. Vitrocéramique selon l'une des revendications précédentes, **caractérisée en ce que** le matériel semi-transparent comprend:
| matériel | pour cent en poids |
|---|---|
| SiO₂ | 68 - 71 |
| Al₂O₃ | 9 - 11 |
| Na₂O | 4 - 5 |
| K₂O | 9 - 10 |
| CaO | 1.5 - 2.5 |
| BaO | 0.5 - 1.5 |
| CeO₂ | 0.5 - 1 |
| TiO₂ | 0.2 - 0.5 |
| B₂O₃ | 0.5 - 2 |

6. Vitrocéramique selon l'une des revendications précédentes, **caractérisée en ce que** un pigment a été additionné au matériel semi-transparent de la vitrocéramique.

7. Vitrocéramique selon la revendication 6, **caractérisée en ce que** le pigment est sélectionné du groupe contenant les chromates, vanadates, manganates et des mélanges de ces matériaux.

8. Vitrocéramique selon l'une des revendications précédentes, **caractérisée en ce que** le coefficient de dilatation thermique est à peu près de 19 • 10⁻⁶ K⁻¹.

9. Un procédé pour produire la vitrocéramique pour la restauration dentaire selon l'une des revendications précédentes, **caractérisé en ce que** des silicates, carbonates ou oxydes sont mélangés en tant que matériaux de départ dans une composition qui est telle que, après l'opération de fusion, ils ont la composition de ladite vitrocéramique semi-transparente, **en ce que** le mélange résultant des matériaux de départ est fondu dans un creuset à environ 1500°C, **en ce que** le verre fondu du four à fusion est goutté directement dans de l'eau, **en ce que**, après le séchage, le matériel de verre solidifié est broyé dans une opération de broyage à sec, et **en ce que** la poudre de verre est vitrifiée et en même temps est attrempée dans des formes souhaitées pour produire les produits et objets de restauration dentaire à base de vitrocéramique ou **en ce que** la poudre de verre est pressée à sec et est converti dans une vitrocéramique solide par une opération de frittage/attrempage.

10. Le procédé selon la revendication 9, **caractérisé en ce que** les produits de la restauration dentaire sont formés dans la forme de couronnes ou d'inlays sur un modèle réfractaire en utilisant une barbotine formée à partir de la poudre de verre et de l'eau et sont vitrifiés et attrempés à environ 820°C pour un temps de maintien de 10 minutes, ou **en ce que** les produits de restauration dentaire sont presses à froid dans un modèle réfractaire à partir d'une masse de verre plastifiée et sont en suite vitrifiés et attrempés sous pression entre environ 800 et 900°C.
